# EUROPEAN PATENT APPLICATION

(11) **EP 3 415 230 A1**
(43) Date of publication of application: **19.12.2018**
(21) Application number: 17176437.6
(22) Date of filing: 16.06.2017
(51) Int. Cl.: B01J 19/00, B01L 3/00, B82Y 40/00, C12M 1/12

(54) **REGULATION OF DROPLET SIZE ON AN ELEMENT POST FABRICATION**

(71) Applicant: Danmarks Tekniske Universitet, 2800 Kgs. Lyngby (DK)
(72) Inventor: Mandsberg, Nikolaj Kofoed, 2800 Kgs. Lyngby (DK); Taboryski, Rafael Jozef, 2880 Bagsværd (DK)
(74) Representative: Plougmann Vingtoft a/s

(57) **Abstract**

The present invention relates to a method for producing liquid droplets of a desired size on specific surface areas of an element, by submerging the element into a liquid reservoir and withdrawing it with a predetermined velocity. The method allows for directional variation in droplet size within the drop pattern, post fabrication, by control of the velocity by which the element is withdrawn from the liquid.

The present invention further relates to a device, which may be used to submerge/withdraw an element to/from a liquid reservoir.

## Description

### FIELD OF THE INVENTION

The present invention relates to a method for producing liquid droplets on elements, such as array chips. The method allows for directional variation in droplet size within the drop pattern, post fabrication, by control of the velocity by which the element is withdrawn from the liquid.

### BACKGROUND OF THE INVENTION

Creating arrays of chemicals, proteins and/or cells is of great interest to the industry, as it allows for simultaneous monitoring of several reactions or parameters. Previously, the pipetting technique has been widely used to create such arrays, but recently methods that are more sophisticated have been developed. One such method is the use of dip-coated biphillic micro-array chips with droplets containing cells/proteins etc. The biphillic micro-array chips may comprise hydrophilic circular spots surrounded by hydrophobic material. When the chips are dipped in a hydrophilic liquid, liquid droplets are adhered to the hydrophilic areas as the chip is withdrawn from the liquid. The parallel droplets can then be analysed. At present, the volume of the droplets is determined before fabrication of the array chips through variations in the hydrophilic area size. However, it would be beneficial if the drop volume could be altered post-fabrication, to reduce the need for production of various hydrophilic spot sizes and to access a wide range of liquid-surface contact angles for a specific spot size.

### OBJECT OF THE INVENTION

An object of the present invention is to provide an alternative to the prior art, by providing a method to produce liquid droplets on an element and regulate the droplet size post fabrication of the element.

### SUMMARY OF THE INVENTION

Thus, the above described object and several other objects are intended to be obtained in a first aspect of the invention by providing a method for producing liquid droplets on specific surface areas of an element, the method comprising: submerging said element with more than one specific surface area, which is either hydrophilic or oleophilic, in a liquid reservoir comprising a liquid, wherein more than one of the specific surface areas are completely covered by the liquid and withdrawing the element from the liquid with at least one velocity, said velocity being defined by a speed and an angle of less than 90 degrees from the surface of the liquid to the element, wherein the speed and the angle are predetermined so as to produce liquid droplets of a desired size.

In one embodiment of the present invention, the surface of the element may be biphilic, comprising either hydrophilic or oleophilic areas as well as either hydrophobic or oleophobic areas, respectively.

In one embodiment of the present invention, the element may be an array chip, such as a micro array chip.

In one embodiment of the present invention, the hydrophilic/oleophilic areas on the surface of said element may be either circular spots or spots in the shape of a polygon with three or more sides.

In one embodiment of the present invention, the element may be withdrawn from the liquid in two or more sequential steps, such as three or four or five or six sequential steps, each step being carried out with predetermined withdrawal speed(s) and withdrawal angle for each step, so as to produce liquid droplets with at least two significantly different volumes, such as three or four or five or six different volumes.

In one embodiment of the present invention, the volume of liquid droplets on any area comprising hydrophilic areas of the surface of said element may be controlled individually, dependent on the angle and the speed by which an area of said element is withdrawn from the liquid reservoir.

In one embodiment of the present invention, the surface of the element may comprise circular hydrophilic/oleophilic areas with a diameter between 1 mm and 20 mm, such as 3 mm and 7 mm.

In one embodiment of the present invention, the droplet volume of the produced liquid droplets may be between 1µL and 1500µL, such as 1µL and 1000µL or 1µL and 500µL.

In one embodiment of the present invention, the height of the produced liquid droplets may be between 0.01 mm and 3 mm, such as 0.05 mm and 2 mm or 0.06 mm and 1.5 mm.

In one embodiment of the present invention, the liquid may comprise DNA and/or proteins and/or chemicals and/or cells and/or molten polymers.

A second aspect of the present invention may be a device comprising a wall element and a liquid reservoir, wherein at least an end of said wall element extends from the liquid reservoir in a longitudinal direction, with an angle between 1 and 180 degrees, such as 10-90 degrees, as defined by gravity, means of controlling said angle by which said wall element extends in a longitudinal direction from the liquid reservoir, a sledge configured to carry an element and a puller attached to the sledge by a line or a wire, so as to: submerge the sledge in the liquid contained in the liquid reservoir and withdrawn the sledge from the liquid contained in the liquid reservoir with a predetermined puller speed.

In one embodiment of the present invention, the sledge may move on a trail formed integral on the wall element in a longitudinal direction, said trail being configured in a way so that the sledge can be withdrawn from the liquid reservoir by a force pulling the sledge in a longitudinal direction along the wall element.

In one embodiment of the present invention, the device may be configured for carrying out the method of the present invention.

In one embodiment of the present invention, the device of the present invention may be used to submerge/withdraw the biphillic element from said liquid reservoir, said specific device comprising a wall element and a liquid reservoir, wherein at least an end of said wall element extends from the liquid reservoir in a longitudinal direction, with an angle between 10 and 90 degrees as defined by gravity, means of controlling said angle by which said wall element extends in a longitudinal direction from the liquid reservoir, a sledge configured to carry a biphillic element and a puller attached to the sledge by a line or a wire, so as to: submerge the sledge in the liquid contained in the liquid reservoir and withdrawn the sledge from the liquid contained in the liquid reservoir with a predetermined puller speed.

In an embodiment of the present invention, a conveyer belt comprising a suitable liquid reservoir with adjustable speed and inclination angle such that withdrawal speed and angle can be programmed, may be used to submerge/withdraw said element from said liquid reservoir.

The first, second and third aspect of the present invention may each be combined with any of the other aspects. These and other aspects of the invention will be apparent from and elucidated with reference to the embodiments described hereinafter.

### BRIEF DESCRIPTION OF THE FIGURES

The method according to the invention will now be described in more detail with regard to the accompanying figures. The figures show one way of implementing the present invention and is not to be construed as being limiting to other possible embodiments falling within the scope of the attached claim set.
Figure 1 illustrates droplet formation on a biphillic element being withdrawn from a liquid reservoir,
Figure 2 shows four biphillic array chips after dip coating, with different patterns and sizes of hydrophilic areas,
Figure 3 illustrates the sample fabrication of a biphillic surface structure nanotextured by reactive ion etching (RIE) and chemically patterned with a hydrophobic FDTS self-assembled monolayer,
Figure 4 illustrates the production of droplets of diverging volume on the same array chip, by changing the speed and the angle by which each row of hydrophilic areas of the array chip is withdrawn from the water,
Figure 5 shows the average droplet mass m on an element, as a function of the withdrawal speed, with a fixed withdrawal angle of 30 degrees. The correlation is plotted for hydrophilic areas with diameters of 3, 5, and 7mm,
Figure 6 shows the average droplet mass m on an element as a function of sine to the withdrawal angel, with a fixed withdrawal velocity of 4.48 cm/s. The correlation is plotted from 24-80 degrees. r²=0.995,
Figure 7. Figure 7a shows the droplet volume as a function of the withdrawal speed for three diameters of circular hydrophilic surface areas, with a fixed withdrawal angle of 30 degrees. Figure 7b shows the droplet volume as a function of the withdrawal speed and the withdrawal angle, with a fixed diameter of the hydrophilic surface areas (3 mm).

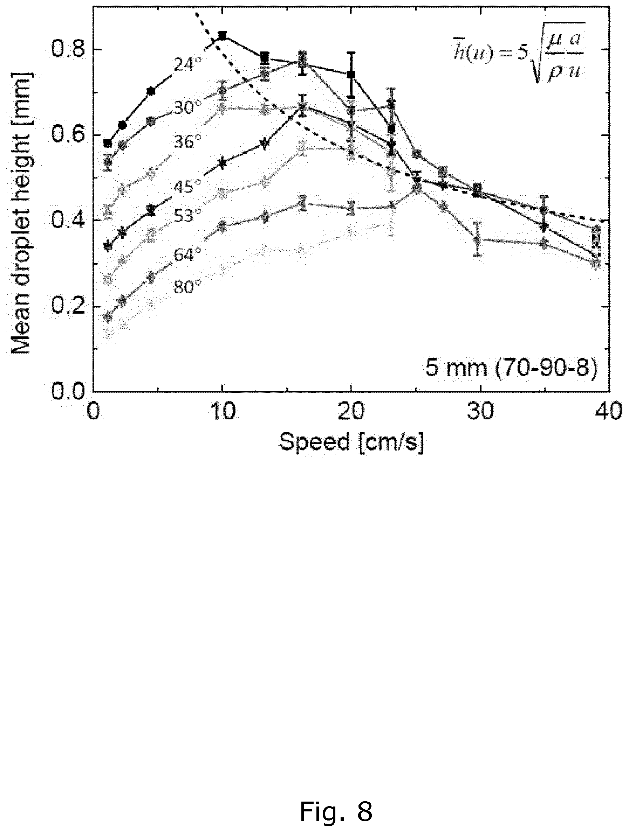

Figure 8 shows a plot of mean droplet height [mm] as a function of speed [cm/s] for seven different withdrawal angles,
Figure 9 schematically illustrates a device according to a preferred embodiment of the invention
and
Figure 10 shows a device according to a preferred embodiment of the invention.

### DETAILED DESCRIPTION OF THE INVENTION

In one aspect, the present invention relates to a method for producing liquid droplets 1 on specific surface areas 2 of an element 4. The method allows for directional variation in droplet size within the droplet pattern, post fabrication, by control of the velocity 6 by which the chip is withdrawn from the liquid 7.

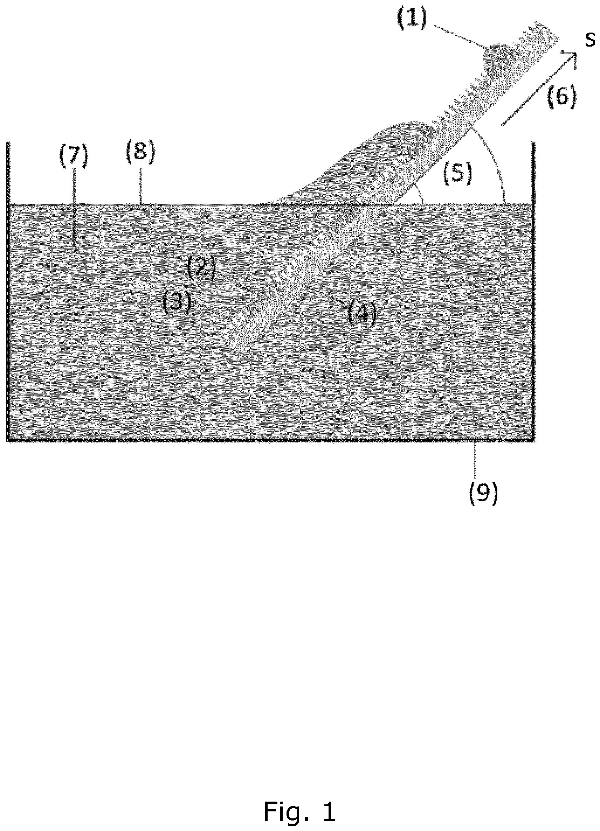

Reference is made to fig. 1, illustrating liquid droplet 1 formation on a biphillic element 4 being withdrawn from a liquid reservoir 9. The biphillic element 4 is withdrawn from the liquid reservoir 9 with a predetermined velocity 6. The biphillic element 4 comprises hydrophilic/oleophilic areas 2 and hydrophobic/oloephobic areas 3. The liquid reservoir comprises a liquid 7. As the biphillic element 4 is withdrawn from the polar liquid 7 with a predetermined velocity 6, liquid droplets 1 of a desired size are produced on the hydrophilic areas 2.

In fluid mechanics, the production of liquid droplets 1 on an element 4 may be referred to as dewetting. The process may occur at a solid-liquid interface, when a thin liquid film on an element ruptures and droplets are produced at random areas of the element surface.

The method of the present invention is a dewetting process wherein liquid droplets 1 are formed on specific surface areas 2 of an element 4. The method of the present invention takes advantage of the wetting properties of different surfaces. A surface with hydrophilic properties attracts water and other polar liquids, whereas a surface with hydrophobic properties repels water and other polar liquids. In the same way, a surface with oleophillic properties attracts oil, whereas an oleophobic surface repels oil.

The general method of the present invention comprises at least two steps:
In the first step, an element 4 comprising more than one specific surface area 2, which is either hydrophillic or oleophillic, is being partially or fully submerged into a liquid reservoir 9 comprising a liquid 7, wherein the more than one of the specific surface areas 2 are completely covered by the liquid 7.
In the second step, the element 4 is partially or fully withdrawn from the liquid 7 with at least one velocity 6. The velocity 6 is predetermined so as to produce liquid droplets 1 of a desired size.

The liquid 7 in the liquid reservoir 9 may preferably be water or oil. The liquid 7 is preferably a solution, comprising DNA and/or proteins and/or chemicals and/or cells and/or molten polymers.

The element 4 may preferably be a biphillic element comprising a biphillic surface comprising hydrophilliv/oleophillic surface areas 2, as well as hydrophobic/oleophobic surface areas.
The surface area surrounding the hydrophilic or oleophilic areas 2 of the element 4 is preferably hydrophobic or oleophobic, respectively. Thus, an element 4 with a biphillic surface comprises hydrophilic or oleophilic areas 2 as well as hydrophobic or oleophobic areas 3, respectively. In this way, the liquid 7 may adhere to the specific surface areas 2 during withdrawal of the element 4 from the liquid reservoir 9 and produce liquid droplets 1 on the element 4.

If the specific surface areas 2 are hydrohpillic areas, the liquid wherein the element 4 is submerged, is preferably a polar liquid 7. In this way, when the biphillic element 4 is withdrawn from the polar liquid 7, liquid droplets 1 are produced on the hydrophilic areas 2.
If specific surface areas 2 are oleophilic areas, the liquid wherein the element 4 is submerged, is preferably an oil. In this way, when the biphillic element 4 is withdrawn from the oil, oil droplets 1 are produced on the oleophilic areas 2.

Surface roughness and chemical heterogeneities can change the wetting properties of an element 4. For example, adding surface topography at the micro or nano scale onto a hydrophobic material, produces super hydrophobicity. Films whose water contact angle is higher than 150° are called superhydrophobic and are fabricated by combining appropriate surface roughness with surfaces of low surface energy. Generally, the rougher the surface, the more hydrophobic it is. Thus, wetting properties can be enhanced by introducing surface roughness. The surface roughness is defined as the true area divided by the projected area of the hydrophilic/oleophillic areas 2.
The surface roughness required for a hydrophobic area 3 of the present invention depends on: spot size, spot shape, roughness topography (the actual shapes of the "blades of silicon"), surface chemistry, liquid type, withdrawal velocity 6, and environmental conditions. The surface roughness required for a hydrophobic area 3 of the present invention may be at least 2.0, such as at least 3 or such as at least 4.
The element 4 may comprise one or more surface areas comprising nanograss. The element 4 may comprise one or more surface areas comprising upright blades of silicon, having a height in the order of 10⁻⁶ m, wherein the design of the surface areas controls the way the surface interacts with liquids.
A hydrophobic surface area of the present invention may be superhydrophobic with a roll-of angle of no more than 30 degrees.
A hydrophilic surface area of the present invention may be superhydrophillic with a contact angle of less than 10 degrees.
The hydrophilic/oleophilic areas 2 on said element 4 pins a polar/oil droplet under vertical conditions preventing fall off. The liquid droplet 1 size may be comparable to the hydrophilic/oleophilic area 2 raised to the power 3/2. The one or more hydrophobic/oleophobic areas 3 on the biphillic element 4 have the ability to shed droplets, e.g. by having a roll-off angle less than 90 degrees for droplet sizes comparable to the hydrophilic/oleophilic area raised to the power 3/2, such as a 1 or 2 or 3 or 5 or 10 or 50 or 100 µL droplet.

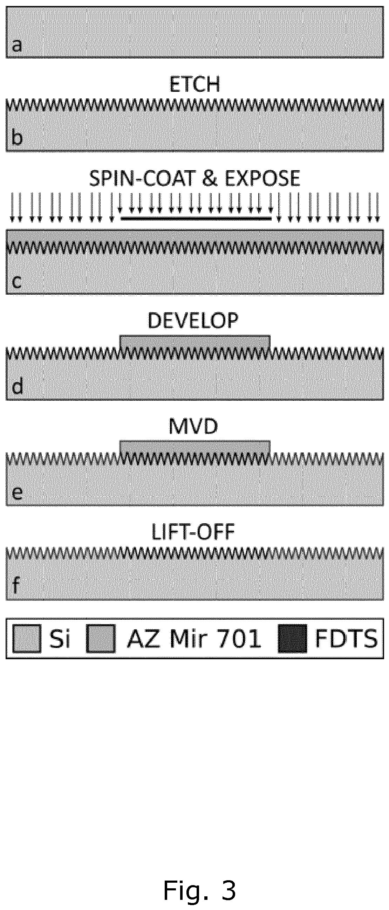

A biphillic surface, comprising hydrophillic/oleophillic areas 2 and hydrophobic/oelophobic areas 3 respectively, may be produced by the method illustrated in fig. 3. Fig. 3 shows the sample fabrication of a biphillic surface structure nanotextured by reactive ion etching (RIE) and chemically patterned with a hydrophobic FDTS self-assembled monolayer (see example section for more details).

The size (volume, height, diameter (for circular areas)) of the produced liquid droplets 1 depends on several factors. Some factors, such as size and shape of the hydrophillic/oleophillic surface areas 2, are determined premanufacturing of the element 4.
The hydrophilic/oleophilic areas 2 on the surface of said element 4 may be in any size or shape. Preferably, the hydrophilic/oleophilic areas 2 are circular. The hydrophilic/oleophilic areas 2 may also be in the shape of a polygon with three or more sides, such as three sides (Triangle), four sides (Square), such as five sides (Pentagon), such as six sides (Hexagon).

The present invention relates to a method for producing liquid droplets 1 on an element, wherein the desired size of the liquid droplets 1 can be determined post-fabrication of the element 4.

The present inventors have found that the liquid droplet 1 size depends on the velocity 6 by which an element 4 is withdrawn from a liquid 7.
The velocity 6 is defined by a speed s and an angle 5. The speed s and angle 5 are predetermined so as to produce liquid droplets 1 of a desired size.
The angle 5 by which said element 4 is withdrawn from the liquid 7 is preferably between 1 and 90 degrees, such as between 5 and 85 degrees, from the surface 8 of the liquid 7 to the element 4. The hydrophilic/oleophilic surface areas 2 are preferably not angled toward the liquid surface 8 during withdrawal of said element 4 from the liquid 7. However, the method may be performed with an overhang, wherein the hydrophilic/oleophilic surface areas 2 are angled towards the liquid surface 8.
The withdrawal speed s by which said element 4 is withdrawn from the liquid 7 is preferably between 0 cm/s and 60 cm/s, such as between 1 cm/s and 45 cm/s.

The droplet volume of the liquid droplets 1 produced by the method of the present invention may be between 1µL and 1500µL, such as 1µL and 1000µL or 1µL and 500µL.

The height of the liquid droplets 1 produced by the method of the present invention may be between 0.01 mm and 3 mm, such as 0.05 mm and 2 mm or 0.06 mm and 1.5 mm.
The diameter of liquid droplets 1 produced on circular surface areas 2 by the method of the present invention may between 1 mm and 20 mm, such as 3 mm and 7 mm.

The hydrophilic/oleophilic areas 2 on the surface of an element 4, may be in a pattern determined premanufacturing of the element 4. This pattern may also be referred to as the droplet pattern, as the droplets 1 will adhere to the surface of an element 4 depending on the pattern of hydrophilic/oleophillic areas 2.
The method of the present invention allows for variation in droplet size within the droplet pattern, post fabrication, by control of the velocity 6 by which the chip is withdrawn from the liquid.

The surface of an element 4 may comprise at least one row of hydrophilic/oleophilic areas 2, wherein the rows may comprise between 1 and 1000 hydrophilic/oleophilic areas 2.
The surface 2, 3 of an element may comprise between 1-1000 parallel rows of hydrophilic/oleophilic areas 2. The parallel rows of hydrophilic/oleophilic areas 2 may all have the same number of hydrophilic/oleophilic areas 2 or the parallel rows of hydrophilic/oleophilic areas 2 each have a number of hydrophilic/oelophilic areas 2, which is not the same between all of the parallel rows 11.

The element 4 may be an array chip, such as a micro array chip.

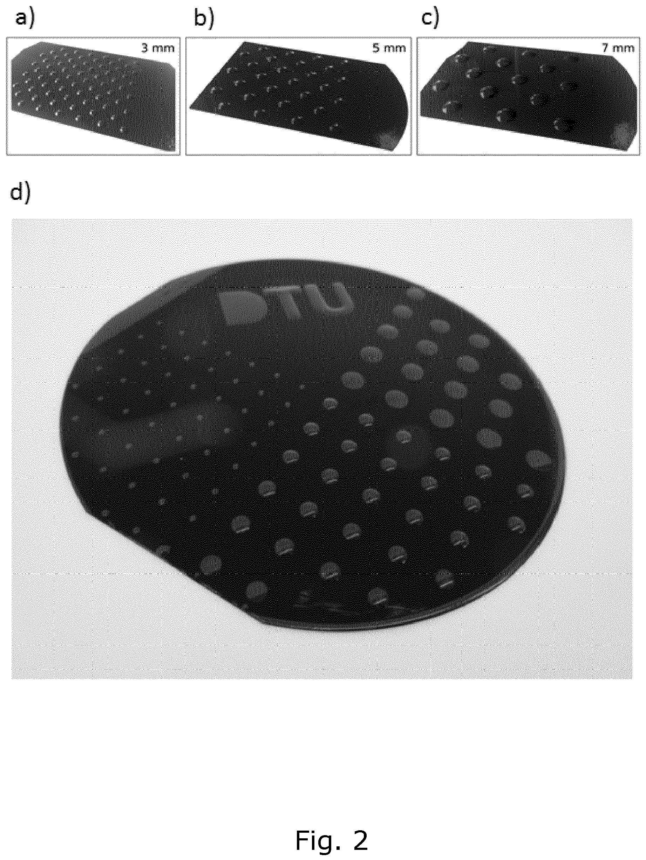

Reference is made to fig. 2 illustrating several biphillic elements 4 according to an embodiment of the present invention. The biphillic elements 4 are array chips, comprising circular hydrophilic surface areas 2, whereon liquid droplets 1 are located, surrounded by a hydrophobic surface area 3.
Fig. 2a illustrates a biphillic micro array chip with a droplet pattern comprising thirteen parallel rows of hydrophilic surface areas 2, wherein each row comprises between six and four hydrophilic surface areas 2. The hydrophilic surface areas 2 are circular with a diameter of 3 mm.

Fig. 2b illustrates a biphillic micro array chop with a droplet pattern comprising eight parallel rows of hydrophilic surface areas 2, wherein each row comprises alternately four and three hydrophilic surface areas 2. The hydrophilic surface areas 2 are circular with a diameter of 5 mm.
Fig. 2c illustrates a biphillic micro array chip with a droplet pattern comprising six parallel rows of hydrophilic surface areas 2, wherein each row comprises alternately two and three hydrophilic surface areas 2. The hydrophilic surface areas 2 are circular with a diameter of 7 mm.
Fig. 2d illustrates a circular biphillic array chip with a droplet pattern comprising three areas of liquid droplets 1 with different sizes of circular hydrophilic surface areas 2.

Depending on the droplet pattern, the volume-velocity correlation may change due to cross-talk between the created droplets 1. This may be exploited to create, further variation in the droplet sizes in the array.

The element 4 may be withdrawn from the liquid 7 in two or more sequential steps, such as three or four or five or six sequential steps, each step being carried out with a predetermined withdrawal speed s and withdrawal angle 5 for each step, so as to produce liquid droplets 1 with at least two significantly different volumes, such as three or four or five or six different volumes.
There might be a smaller natural variation in droplet size on an area comprising more than one specific surface areas 2, when the whole area is withdrawn from the liquid with one predetermined velocity 6. By significantly different volumes, it is meant that the variation in size between liquid droplets 1 are bigger than the standard variation between droplets of an area withdrawn from the liquid 7 with one predetermined velocity 6.

The volume of liquid droplets 1 on any area comprising hydrophilic areas 2 of the surface of said element 4 may be controlled individually, dependent on the angle 5 and speed s by which an area of said element 4 is withdrawn from the liquid reservoir 9.

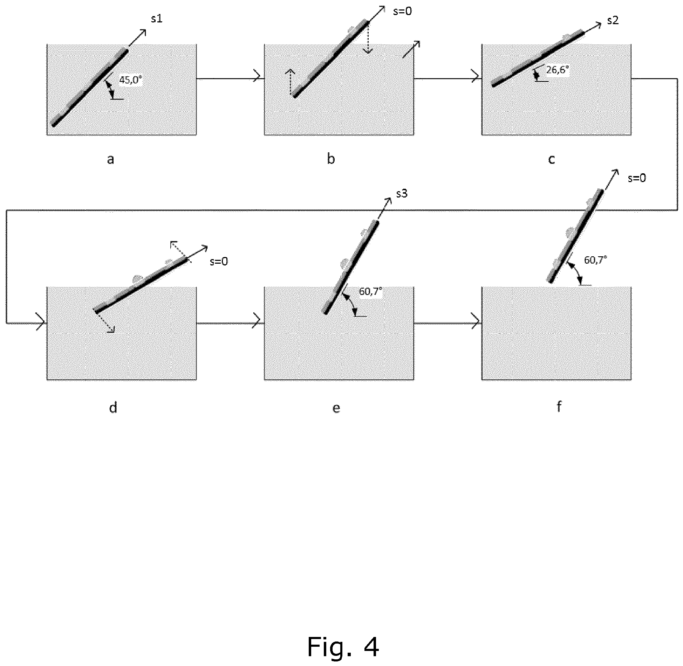

Reference is made to fig. 4 illustrating an element 4 being submerged and withdrawn from a liquid 7 in three sequential steps, producing liquid droplets 1 with three different sizes.

In fig. 4a, an element 4 comprising three hydrophilic surface areas 2 has been fully submerged in a liquid reservoir 9 comprising a polar liquid 7. In a first sequential step, the element 4 is placed in a predetermined withdrawal angle 5 of 45 degrees, in a way so that the hydrophilic surface areas 2 are not faced toward the bottom of the liquid reservoir 9. An area of the element 4 comprising the first hydrophilic surface area 2 is to be withdrawn by a predetermined withdrawal speed s1 from the liquid reservoir. In fig. 2b a first area of the element 4 has been withdrawn from the liquid 7 and a first liquid droplet 1 has been produced on the element 4. In a second sequential step, the element 4 is being tipped into a new angle (as defined by dotted arrows) without withdrawing more of the element 4 from the liquid (s=0). Fig. 4c illustrates when the element 4 has been placed in a new predetermined withdrawal angle and a second area of the element 4 comprising the second hydrophilic surface area 2 is withdrawn from the liquid with a predetermined speed (s2) to produce a second liquid droplet 1 with a size significantly different from the first liquid droplet 1.

Fig. 4d is an illustration of the third sequential step, wherein the element 4 is being tipped into a new angle (as defined by dotted arrows) without withdrawing more of the element 4 from the liquid (s=0). Fig. 4e illustrates when the element 4 has been placed in a new predetermined withdrawal angle and a third area of the element 4 comprising the third hydrophilic surface area 2 is withdrawn from the liquid with a predetermined speed (s3) to produce a third liquid droplet 1 with a size significantly different from the first and second liquid droplets 1.

Fig. 4f illustrates an element 4 comprising three different droplets with significantly different volumes.

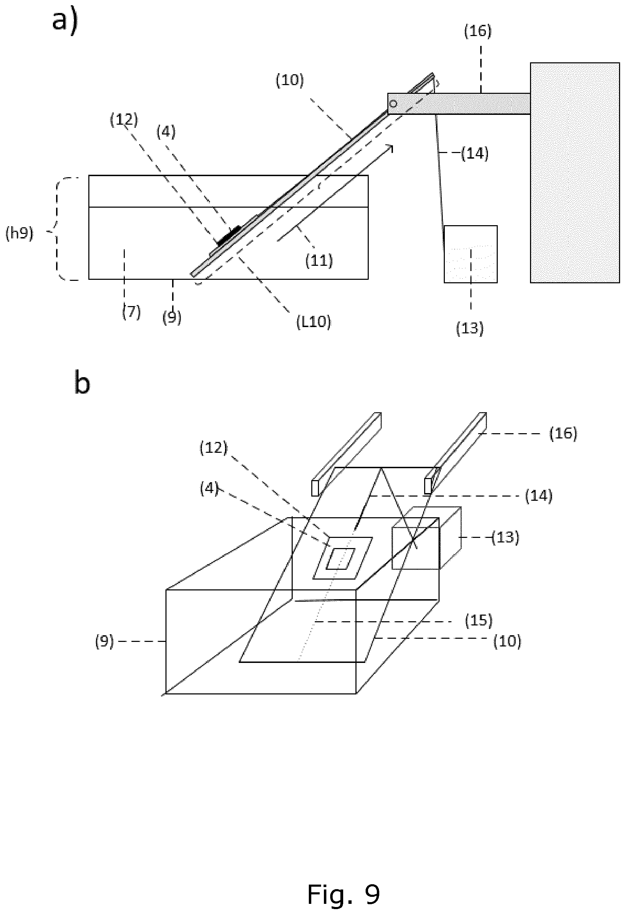

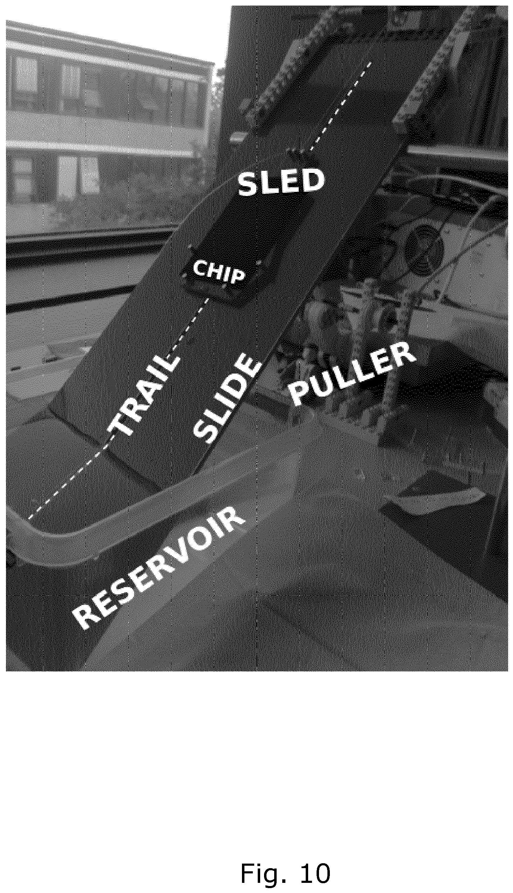

The present invention further relates to a device for submerging/withdrawing an element 4 from a liquid reservoir 9. Reference is made to figs. 9 and 10, illustrating an embodiment of a device of the present invention.

The device comprises a wall element 10 and a liquid reservoir 9, wherein at least an end of said wall element 10 extends from the liquid reservoir 9 in a longitudinal direction 11, with an angle 5 between 1 and 180 degrees, such as 10-90 degrees, as defined by gravity. The device further comprises means 16 of controlling said angle 5 by which said wall element 10 extends in a longitudinal direction 11 from the liquid reservoir 9. The device also comprises a sledge 12 configured to carry an element 4 and a puller 13 attached to the sledge 12 by a line or a wire 14, so as to submerge the sledge in the liquid contained in the liquid reservoir 9 and withdrawn the sledge from the liquid 7 contained in the liquid reservoir 9 with a predetermined puller speed. The predetermined puller speed may be determined by the motor power settings and the chosen gearing of the puller.
The sledge may move on a trail 15 formed integral on the wall element 10 in a longitudinal direction 11, said trail 15 being configured in a way so that the sledge can be withdrawn from the liquid reservoir 9 by a force pulling the sledge 12 in a longitudinal direction 11 along the wall element 10.

The height of the liquid reservoir should be larger than the element length (L4) times sine to the withdrawal angle 5, plus the distance it takes for the specific puller to reach the desired pulling speed.
Reservoir height > sine(angle)*length(element) + distance it takes for the specific puller to reach the desired pulling speed.

The wall element 10 and sledge 12 may preferably primarily be of a water-resistant and/or corrosion-resistant material, such as glass, plastic or stainless steel.

The device of the present invention may be used for dewetting of a biphillic element 4 and/or configured for carrying out the method of the present invention.

The present invention further relates to a method, wherein a device according to the present invention is used to submerge/withdraw a biphillic element 4 from a liquid reservoir 9. The device comprises a wall element 10 and a liquid reservoir 9, wherein at least an end of said wall element 10 extends from the liquid reservoir 9 in a longitudinal direction 11, with an angle 5 between 1 and 180 degrees, such as 10-90 degrees, as defined by gravity. The device further comprises means 16 of controlling said angle 5 by which said wall element 10 extends in a longitudinal direction 11 from the liquid reservoir 9. The device also comprises a sledge 12 configured to carry an element 4 and a puller 13 attached to the sledge 12 by a line or a wire 14, so as to: submerge the sledge in the liquid contained in the liquid reservoir 9 and withdrawn the sledge from the liquid 7 contained in the liquid reservoir 9 with a predetermined puller speed. The predetermined puller speed may be determined by the motor power settings and the chosen gearing of the puller. The sledge may move on a trail 15 formed integral on the wall element 10 in a longitudinal direction 11, said trail 15 being configured in a way so that the sledge can be withdrawn from the liquid reservoir 9 by a force pulling the sledge 12 in a longitudinal direction 11 along the wall element 10.

An industrial robot comprising a suitable griper may be used to submerge/withdraw the element 4 from the liquid reservoir 9.
A conveyer belt comprising a suitable liquid reservoir 9 with adjustable speed and inclination angle such that withdrawal speed and angle can be programmed, may be used to submerge/withdraw the element 4 from said liquid reservoir 9.
The method of submerging/withdrawing said element from the liquid may be dip-coating or plate coating.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

### LIST OF REFERENCE SYMBOLS USED

- 1: Liquid droplet
- 2: Hydrophilic/oleophilic area (specific surface area)
- 3: Hydrophobic/oleophobic area
- 4: Element, preferably a biphillic element comprising both hydrophilic and hydrophobic surface areas.
- 5: Withdrawal angle, as defined by gravity
- 6: Withdrawal velocity, defined by withdrawal speed (s) and angle (5)
- 7: Liquid
- 8: Liquid surface
- 9: Liquid reservoir
- s: withdrawal speed
- s1: speed 1
- s2: speed 2
- s3: speed 3
- *m*: mass of liquid droplet (Volume)
- h4: height of element
- h9: height of liquid reservoir
- L10: length of wall element
- 10: wall element
- 11: longitudinal direction
- 12: sledge
- 13: puller
- 14: line or wire
- 15: trail
- 16: means of controlling the angle by which the wall element longitudinal extends from the liquid reservoir

### EXAMPLES

### Preparation of biphillic array chip (photo mask):

A photo mask was prepared by printing three times on top of each other on Premium Transparencies from Xerox with a Xerox 7502V_U printer using the bypass feeder. An array of non-transparent circles/spots of diameter d on the photomask defines the regions of hydrophilicity. The spots were organized in a square array aligned with respect to the direction of withdrawal.

### Fabrication of biphillic micro array chip

Figure 3 (a-f) shows a schematic representation of the fabrication process flow for making the array chips. Biphilic surfaces were achieved by means of a lithographic process whereby a nanotextured silicon wafer with a native oxide surface was coated with a monolayer of a hydrophobic fluorocarbon agent in specific regions. Uncoated regions remained hydrophilic. Thus, both regions, hydrophilic and hydrophobic, had the same uniform nanotexture that enhanced their chemical properties. P-type 100 mm (100) silicon wafers were used for the fabrication (Figure 3a). Reactive ion-etching (RIE, Pegasus D-RIE, STS, UK) was initially used to create a nanotexturing, frequently referred to as nanograss (Figure 3b). The nanograss recipe from Schneider et al.¹⁰ was used and comprised a mixture of either 90 or 50 sccm O₂ and 70 sccm SF₆ gas for 8 minutes; the resulting nanograss structures are denoted 70-90-8 and 70-50-8 surfaces, respectively. The recipes are reported to give different roughness factors (actual surface area to projected surface area) for the two different O₂ flows as listed in Table 1. The wafers were then treated with hexamethyldisilazane (HMDS) to enhance photoresist adhesion, and subsequently spin coated (Suss MicroTec Gamma 2M spin coater) with 1.5 µm positive tone photoresist (AZ Mir 701). The resist was soft baked at 90 °C for 60 seconds. Afterwards, the resist was exposed for 30 s at an intensity of 7.0 mW/cm² on a mask aligner (SUSS MA6) in flood exposure mode through a photo mask placed directly on top of the wafer. The resist was then baked at 110 °C for 60 seconds and developed in AZ 726 MIF for 60 seconds (Suss MicroTec Gamma 2M) (Figure 2d). Finally, Figure 2e shows deposition of a self-assembled hydrophobic monolayer using the precursor perfluorodecyltrichlorosilane (FDTS) in molecular vapour deposition (MVD, MVD 100, MST, USA), and Figure 2f shows lift-off in acetone for 7 minutes followed by a 5-minute rinse in DI water.

**Table 1**

| Structure | Roughness Factor* | Hydrophilic *θ_{A}* [°] | Hydrophilic *θ_{R}* [°] | Hydrophobic *θ_{A}* [°]* | Hydrophobic *θ_{R}* [°]* | Hydrophobic Roll-off [°]* |
|---|---|---|---|---|---|---|
| 70-50-8 | 3.2 | 114.1±1.2 | 6.4±1.0 | 167.3±3.0 | 166.1±3.3 | 0.9±0.3 |
| 70-90-8 | 4.0 | 2.7±0.5 | ≤ 2.7±0.5 ** | 159.1±2.1 | 117.5±1.3 | 27.5±0.9 |

| | | | | | | |
|---|---|---|---|---|---|---|
| * Data from reference **Value based on the corresponding advancing contact angle always being larger than the receding | | | | | | |

### Example 1:

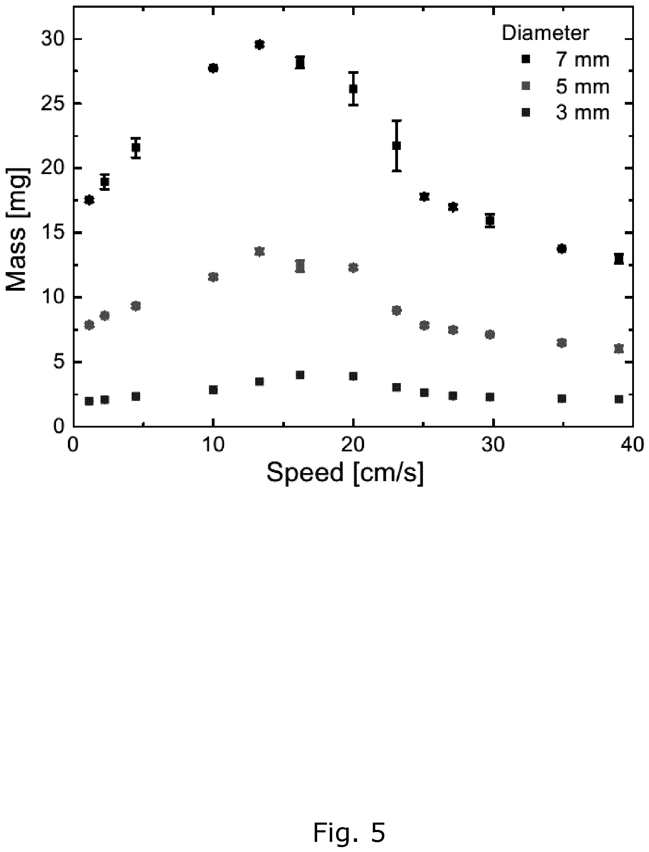

This example investigates the relationship between withdrawal speed and volume of adhered liquid droplets on the hydrophilic surface areas on a biphillic array chip. The withdrawal angle was fixed at 30 degrees.
Naturally, the droplet volume (mass) increases with hydrophilic spot diameter (3, 5 and 7 mm was tested). Fig. 5 shows that the droplet volume increases with withdrawal velocity until a certain point, whereafter the droplet volume drops again. The data suggest that the droplet volume can be increased with at least a factor 2 (from lowest tested speed to highest tested speed) by changing the withdrawal speed.

### Example 2:

This example investigates the relationship between droplet volume of adhered liquid droplets on the hydrophilic surface areas on a biphillic array chip and withdrawal angle. The withdrawal speed (4.48 cm/s) and the hydrophilic spot diameter (5mm) were fixed.

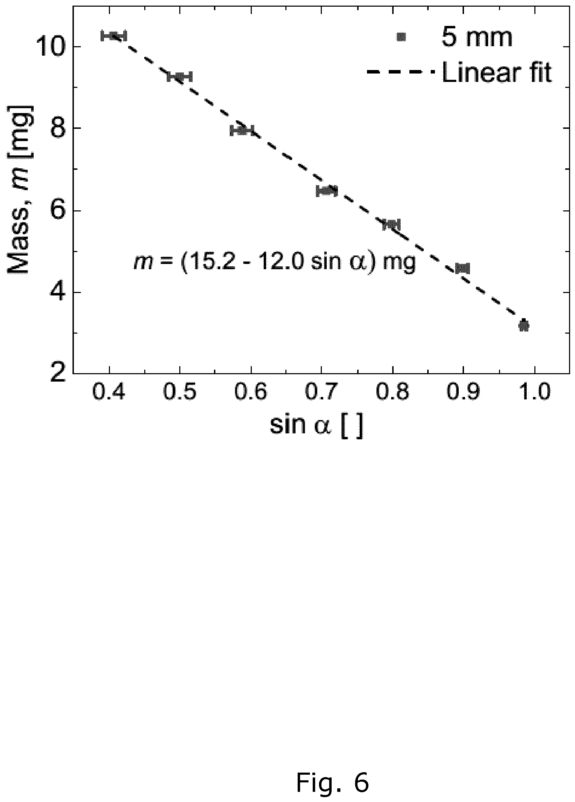

Fig. 6 shows that the droplet volume (mass) increases with a lower angle. The data suggest that the droplet volume can be increased by at least a factor 2 (from the lowest angle to the highest angle) by changing the withdrawal angle.

### Example 3:

In this example, the relationship between droplet volume, withdrawal angel, and withdrawal speed was investigated at a hydrophilic spot diameter of 3, 5 and 7 mm.

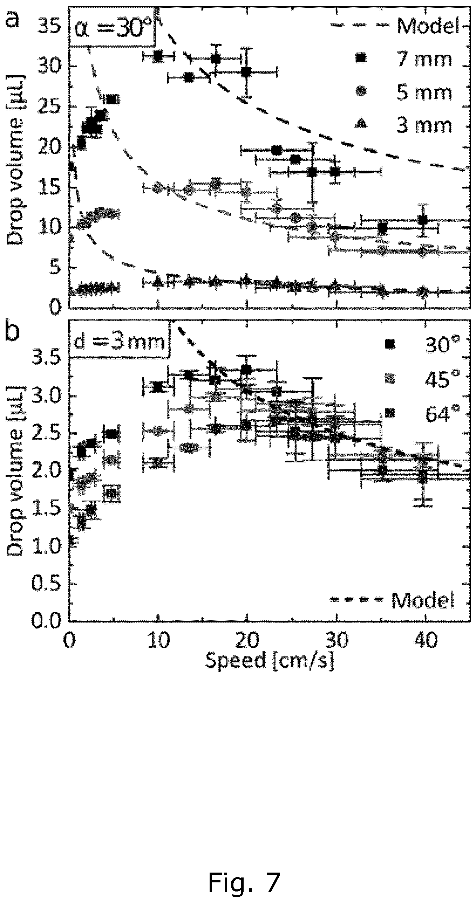

Fig. 7b suggests that the droplet volume increases with speed until a certain point (around 16 cm/s), whereafter it starts to decrease. Furthermore, the droplet volume is generally higher with a low withdrawal angle (30 degrees) compared to a higher withdrawal angle (64 degrees). As it can be seen from fig 7a, the variance that can be obtained by changing the withdrawal speed and angle, will only get bigger as the diameter of hydrophilic surface areas is increased.

### Example 4:

In this example, the relationship between droplet height, withdrawal speed, and withdrawal angle is investigated. The diameter of hydrophilic spots is fixed at 5 mm. Fig. 7 shows that mean droplet height increases as the withdrawal angle decrease. The mean droplet height increases with withdrawal speed until a certain point (around 20 cm/s), whereafter it decrease. The data in fig. 3 suggest that droplet height can be increased with at least a factor 3 by changing withdrawal speed and withdrawal angle.

## Claims

1. A method for producing liquid droplets (1) on specific surface areas (2) of an element (4), the method comprising:
a) submerging said element (4) with more than one specific surface area (2), which is either hydrophilic or oleophilic, in a liquid reservoir (9) comprising a liquid (7), wherein more than one of the specific surface areas (2) are completely covered by the liquid,
b) withdrawing the element (4) from the liquid (7) with at least one velocity (6), said velocity (6) being defined by a speed (s) and an angle (5) of less than 90 degrees from the surface of the liquid (8) to the element (4), wherein the speed (s) and the angle (5) are predetermined so as to produce liquid droplets (1) of a desired size.

2. The method according to claim 1, wherein the surface of said element (4) is biphilic comprising either hydrophilic or oleophilic areas (2) as well as either hydrophobic or oleophobic areas (3), respectively.

3. A method according to any of claims 1 or 2, wherein said element (4) is an array chip, such as a micro array chip.

4. The method according to any of the preceding claims, wherein the hydrophilic/oleophilic areas (2) on the surface of said element (4) are either circular spots or spots in the shape of a polygon with three or more sides.

5. The method according to any of the preceding claims, wherein said element (4) is withdrawn from the liquid (7) in two or more sequential steps, such as three or four or five or six sequential steps, each step being carried out with a predetermined withdrawal speed (s) and withdrawal angle (5) for each step, so as to produce liquid droplets (1) with at least two significantly different volumes, such as three or four or five or six different volumes.

6. The method according to any of the preceding claims, wherein the volume of liquid droplets (1) on any area comprising hydrophilic areas (2) of the surface of said element (4) is controlled individually, dependent on the angle (5) and the speed (s) by which an area of said element (4) is withdrawn from the liquid reservoir (9).

7. The method according to any of the preceding claims, wherein the surface of said element (4) comprises circular hydrophilic/oleophilic areas (2) with a diameter between 1 mm and 20 mm, such as 3 mm and 7 mm.

8. The method according to any of the preceding claims, wherein the droplet volume of the produced liquid droplets (1) is between 1µL and 1500µL, such as 1µL and 1000µL or 1µL and 500µL.

9. The method according to any of the preceding claims, wherein the height of the produced liquid droplets (1) is between 0.01 mm and 3 mm, such as 0.05 mm and 2 mm or 0.06 mm and 1.5 mm.

10. The method according to any of the preceding claims, wherein said liquid (7) comprises DNA and/or proteins and/or chemicals and/or cells and/or molten polymers.

11. A device comprising:
- a wall element (10) and a liquid reservoir (9), wherein at least an end of said wall element (10) extends from the liquid reservoir (9) in a longitudinal direction (11), with an angle (5) between 1 and 180 degrees, such as 10-90 degrees, as defined by gravity
- means (16) of controlling said angle (5) by which said wall element (10) extends in a longitudinal direction (11) from the liquid reservoir (9)
- a sledge (12) configured to carry an element (4) and a puller (13) attached to the sledge (12) by a line or a wire (14), so as to:
a) submerge the sledge in the liquid contained in the liquid reservoir (9)
b) withdrawn the sledge from the liquid contained in the liquid reservoir (9) with a predetermined puller speed.

12. A device according to claim 11, wherein said sledge moves on a trail (15) formed integral on the wall element (10) in a longitudinal direction (11), said trail (15) being configured in a way so that the sledge can be withdrawn from the liquid reservoir (9) by a force pulling the sledge (12) in a longitudinal direction (11) along the wall element (10).

13. A device according to any of claims 11 or 12, wherein the device is being configured for carrying out the method according to any of the preceding claims 1-10.

14. A method according to any of claims 1-10, wherein a device according to claims 11 or 12 is used to submerge/withdraw the biphillic element (4) from said liquid reservoir (9), said specific device comprising:
- a wall element (10) and a liquid reservoir (9), wherein at least an end of said wall element (10) extends from the liquid reservoir (9) in a longitudinal direction (11), with an angle (5) between 10 and 90 degrees as defined by gravity
- means (16) of controlling said angle (5) by which said wall element (10) extends in a longitudinal direction (11) from the liquid reservoir (9)
- a sledge (12) configured to carry a biphillic element (4) and a puller (13) attached to the sledge (12) by a line or a wire (14), so as to:
a) submerge the sledge in the liquid contained in the liquid reservoir (9)
b) withdrawn the sledge from the liquid contained in the liquid reservoir (9) with a predetermined puller speed.

15. A method according to any of claims 1-10 or 14, wherein a conveyer belt comprising a suitable liquid reservoir with adjustable speed and inclination angle such that withdrawal speed and angle can be programmed, is used to submerge/withdraw said element (4) from said liquid reservoir (9).
